# FASCICULE DE BREVET EUROPEEN

(11) **EP 0 909 556 B1**
(45) Date de publication et mention de la délivrance du brevet: **15.10.2003**
(21) Numéro de dépôt: 98402055.2
(22) Date de dépôt: 13.08.1998
(51) Int. Cl.: A61K 7/48, A61K 7/06, A61K 35/78

(54) **Extrait de Rosacées en tant qu'antagoniste de bradykinine**
Rosaceae extrakt als bradykinin-antagonist
Rosaceae extract as bradykinin antagonist

(30) Priorité: 22.09.1997 FR 9711762
(43) Date de publication de la demande: 21.04.1999
(73) Titulaire: L'OREAL, 75008 Paris (FR)
(72) Inventeur: Breton, Lionel, 78000 Versailles (FR); Pineau, Nathalie, 86000 Poitiers (FR)
(74) Mandataire: Tezier Herman, Béatrice

(56) Documents cités:
- EP-A- 0 727 217
- EP-A- 0 781 544
- FR-A- 2 719 473
- GB-A- 2 095 553
- DATABASE WPI Week 9139 Derwent Publications Ltd., London, GB; AN 91-284722 XP002062719 & JP 03 188013 A (SHISEIDO CO)
- DATABASE WPI Week 9139 Derwent Publications Ltd., London, GB; AN 91-284717 XP002062720 & JP 03 188008 A (SHISEIDO CO)

## Description

L'invention a pour objet l'utilisation, à titre de principe actif, dans une composition d'au moins un extrait d'au moins un végétal de la famille des Rosacées en tant qu'antagoniste de bradykinine. L'invention a en outre pour objet l'utilisation, à titre de principe actif dans une composition d'au moins un extrait d'au moins un végétal de la famille des Rosacées, l'extrait ou la composition étant destinés à traiter le désordres liés à un excès de synthèse et/ou de libération de bradykinine.

La bradykinine est un peptide d'origine plasmatique libéré à partir d'un précurseur kininogène par une protéase plasmatique du nom de kallikreine (EC 3.4.21.24). Ce nanopeptide est un des médiateurs clés de l'inflammation et possède des propriétés mitogènes. Les récepteurs pour cette kinine se divisent en deux principaux sous types, B1 et B2. La bradykinine agit notamment, sur le récepteur B2 et provoque la stimulation de nombreux systèmes de production de seconds messagers dont l'inositol-triphosphates (ip3), connu pour provoquer la libération de calcium à partir des sites de stockages intracellulaires dans les cellules dont le kératinocyte. La bradykinine induit également la phosphorylation des résidus tyrosine par l'intermédiaire d'une activation du récepteur B2, la dépolarisation ou l'hyperpolarisation de la membrane cellulaire ainsi que l'activation du métabolisme de l'acide arachidonique.

La bradykinine est impliquée dans un grand nombre de désordres physiopathologiques comme par exemple la vasodilatation et l'augmentation de la perméabilité vasculaire, l'hypotension, la douleur ou encore la prolifération du tissu conjonctif. La vasodilatation et l'augmentation de la perméabilité vasculaire peuvent avoir comme conséquence, entre autres, la couperose, la télangiectasie ou encore un rhynophyma. La prolifération du tissu conjonctif aboutit à la formation de chéloides.
La bradykinine est soupçonnée de jouer un rôle dans les processus conduisant à la chute des cheveux.

Par ailleurs la bradykinine provoque un processus inflammatoire par activation de la phospholipase A2 et par formation de prostaglandines et/ou la libération de différents médiateurs de l'inflammation : cytokines, leukotriènes. Elle intervient aussi dans la stimulation des terminaisons nerveuses, la contraction des muscles lisses des tractus digestif et respiratoire et de l'utérus, Une production excessive de bradykinine peut provoquer des diarrhées dans les désordres gastro-intestinaux et stimuler les sécrétions nasopharyngiennes dans les rhinites allergiques.

La bradykinine est par ailleurs connue pour stimuler le transport ionique et les sécrétions fluidiques par différents épithélium.

L'utilisation d'antagonistes bradykininergiques est donc l'une des alternatives efficaces à tous les désordres cosmétiques et/ou pathologiques mettant en jeu les systèmes décrits ci-dessus, notamment les processus d'inflammation d'origine neurogène ou non.

Pour qu'une substance soit reconnue comme un antagoniste de la bradykinine elle doit induire une réponse pharmacologique cohérente incluant ou non sa fixation au récepteur de la bradykinine.
Ainsi, entre dans cette définition tout composé qui peut interférer avec les effets de la bradykinine par sa fixation au récepteur de celle-ci (B1 ou B2) et/ou tout composé qui indépendamment de la fixation au(x) récepteur(s) induira par un mécanisme quelconque un effet contraire à celui connu de la bradykinine (par exemple interférant avec la synthèse de la bradykinine).

Particulièrement, pour qu'une substance soit reconnue comme un antagoniste réceptoriel de bradykinine, elle doit répondre notamment, aux caractéristiques suivantes :
- avoir une affinité sélective pour les récepteurs spécifiques de ce composé :
   de type B₁ et/ou B₂.
      Les modèles expérimentaux utilisés sont réalisés sur culture de cellules de l'aorte mésentérique (fixation réceptorielle sur récepteurs B₁ selon la méthode décrite par J.P. Galizzi publiée dans Brit. J. Pharmacol, 1994, 113, 389) et/ou sur intestin (fixation réceptorielle sur récepteurs B₂.selon la méthode décrite par Burch R.M. publiée dans Biotech. Update (Dupont-Nen),1992, 7, 2.
- avoir une activité pharmacologique antagoniste réceptoriel de bradykinine, c'est-à-dire induire une réponse pharmacologique cohérente dans des tests spécifiques.
L'activité pharmacologique est dans ce cas, évaluée sur organes isolés selon la méthode décrite par Rhaleb N.E. et al. dans Brit. J. Pharmacol., 1990, 94, 445 en ce qui concerne une activité antagoniste de type B₁ et/ou B₂.

De nombreux composés ont déjà été décrits comme antagoniste de bradykinine. On peut à cet égard citer des peptides, synthétiques ou naturels, éventuellement modifiés, comme la D-Arg-[Hyp3, D-Phe7]-bradykinin (NPC567), la [Thi 5, 8, D-Phe7]-bradykinin, la D-Arg, [Hyp3, Thi5,8, D-Phe7]-bradykinin, la N-α-adamantaneacetyl-D-Arg-[Hyp3, Thi5,8, D-Phe7]-bradykinin, la des-Arg9, [Leu8]-bradykinin (tous vendus par la société Sigma) ou encore les composés cités dans les brevets WO 95/08566, WO 95/07294, EP 0623350, EP 0622361, WO 94/11021, EP 0596406, WO 94/06453, WO 94/09001, EP 0578521, EP 0564972, EP 0552106, WO 93/11789, US 5216165, US 5212182, WO 92/17201, EP 0496369, EP 0472220, EP 0455133, WO 91/09055, WO 91/02746, EP 0413277, EP 0370453, EP 0359310, WO 90/03980, WO 89/09231, WO 89/09230, WO 89/01780, EP 0334244, EP 0596406, WO 86/07263 ou la P-guanidobenzoyl-[Hyp3,Thi5,D-Tic7,Oic8]-bradyklnin (S 16118) (Feletou M & al., Pharmacol. Exp. Ther., June 1995, 273, 1078-84), la D-Arg-[Hyp3, Thi5, D-Tic7,Oic8]-bradykinine (HOE 140) (Feletou M & al., Eur. J. Pharmacol. 1995, 274, 57-64), la D-Arg-[Hyp3, D-Hype (trans-propyl)7 Oic8]-bradykinin (NPC 17731) ( Herzig M.C.S. and Leeb-Lundberg L.M.F., J. Biol. Chem. 1995, 270, 20591-20598) ou ceux cités dans Bradykinin Antagonists : development and applications (Stewart J.M., Biopolymers, 1995, 37, 143-155), ou encore des molécules chimiques, synthétiques ou naturelles, comme par exemple celles décritent dans Salvino et coll. J. Med. Chem., 1993, 36, 2583-2584.

En règle générale ces antagonistes de bradykinine, qui par ailleurs donnent d'excellent résultats, sont des molécules chimiques qui peuvent présenter des effets secondaires néfastes.

Il reste donc toujours intéressant de pouvoir disposer de nouveaux produits répondant aux critères d'antagoniste de bradykinine, surtout s'ils sont d'origine naturelle et ne présentent pas d'effets secondaires.

A la connaissance de la demanderesse il n'a jamais été démonté ni suggéré dans l'art antérieur qu'un extrait d'au moins un végétal de la famille des Rosacées présente les critères d'antagoniste de bradykinine.

Les plantes de la famille des Rosacées sont surtout utilisées pour leur propriétés aromatiques et ornementales.
Dans l'art antérieur les végétaux de la famille des Rosacées sont connus dans des compositions pour le traitement de maladies urogénitales (FR 2372626), dans des compositions cosmétiques éclaircissantes (JP08208451) ou de protection contre les ultraviolets (EP-A-781544), pour la préparation de composés antioxydants (EP-A-657169), ou encore pour la préparation de composés antimicrobiens et/ou insecticides pour la protection des plantes (DE4327792).

GB 2095553 décrit un procédé de fabrication de compositions pour traiter ou protéger la peau, contenant un mélange d'extraits de végétaux de la famille des Caesalpiniaceae, des Fagaceae, des Rosaceae et/ou des Chemopodiaceae.

FR 2 719 473 concerne un procédé pour accélérer le renouvellement cellulaire de la peau tout en maintenant son hydratation, qui comprend l'application d'un jus de fruit de rosacée dans une base cosmétique appropriée.

EP727217 a pour objet un inhibiteur de hyaluronidase, qui peut notamment être un extrait de Rosaceae.

JP 03188013 décrit un tonique capillaire utile comme inhibiteur de 5 α- réductase contenant un extrait de certains végétaux de la famille des Rosaceae associé à de nombreux autres extraits végétaux.

JP 03188008 se rapporte à des compositions cosmétiques contenant un mélange d'extraits de Rosaceae pour lisser et hydrater la peau.

Mais à la connaissance de la demanderesse l'activité antagoniste de bradykinine d'un extrait d'au moins un végétal de la famille des Rosacées n'a jamais été décrite.

La demanderesse a découvert qu'un extrait d'au moins un végétal de la famille des Rosacées répond aux caractéristiques définies comme caractérisant un antagoniste de bradykinine et peut donc être utilisé comme antagoniste de bradykinine.

L'invention a donc pour objet l'utilisation, à titre de principe actif dans une composition d'un extrait de Rosacées comme antagoniste de bradykinine.

Par principe actif, on entend toute molécule ou extrait susceptible de modifier ou de moduler le fonctionnement d'au moins un système biologique donné.

L'extrait d'au moins un végétal de la famille des Rosacées peut être tout extrait préparé à partir de tout matériel végétal issu d'au moins un végétal de la famille des Rosacées.

Ainsi, l'extrait d'au moins un végétal de la famille des Rosacées utilisé selon l'invention peut être obtenu à partir de matériel végétal issu de plante entière ou de partie de plante comme les feuilles, les tiges, les fleurs, les pétales, les racines ou encore des cellules indifférenciées.

Par cellules végétales indifférenciées, on entend toute cellule végétale ne présentant aucun des caractères d'une spécialisation particulière et capable de vivre par elle-même et non en dépendance avec d'autres cellules. Ces cellules végétales indifférenciées sont éventuellement aptes, sous l'effet d'une induction, à toute différenciation conforme à leur génome.
Selon la méthode de culture choisie, et en particulier selon le milieu de culture choisi, il est possible d'obtenir, à partir d'un même explant, des cellules végétales indifférenciées présentant des caractères différents.

Préférentiellement selon l'invention on utilise des pétales.

L'extrait d'au moins un végétal de la famille des Rosacées peut être tout extrait préparé à partir de tout matériel végétal issu d'au moins un végétal de la famille des Rosacées cultivé *in vivo* ou issu de culture *in vitro*.

Par culture in vivo on entend toute culture de type classique c'est à dire en sol à l'air libre ou en serre, ou encore hors sol.

Par culture *in vitro*, on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo.*

Préférentiellement selon l'invention on utilise un végétal issu de culture *in vivo.*

Toute méthode d'extraction connue de l'homme du métier peut être utilisée pour préparer l'extrait contenu dans la composition selon l'invention.

On peut en particulier citer les extraits aqueux, alcooliques ou utilisant un solvant organique.
Par solvant aqueux on entend tout solvant constitué totalement ou pour part d'eau. On peut citer ainsi l'eau elle-même, les solvant hydroalcooliques en toute proportion ou encore les solvants constitués d'eau et d'un composé comme le propylène glycol en toute proportion.

Parmi les solvants alcooliques on peut citer notamment l'éthanol.

On peut également utiliser un extrait préparé par la méthode décrite dans la demande de brevet français n° 95-02379 déposée par la demanderesse.
Ainsi, dans une première étape on broie le matériel végétal dans une solution aqueuse à froid, dans une deuxième étape les particules en suspension sont éliminées de la solution aqueuse issue de la première étape, et dans une troisième étape on stérilise la solution aqueuse issue de la deuxième étape. Cette solution aqueuse correspond à l'extrait.
D'autre part, la première étape peut avantageusement être remplacée par une opération de congélation simple des tissus végétaux (par exemple à -20°C), suivie d'une extraction aqueuse reprenant les deuxièmes et troisième étapes ci-dessus décrites.

Quel que soit le mode de préparation utilisé selon l'invention des étapes subséquentes visant à favoriser la conservation et/ou la stabilisation peuvent être ajoutées sans pour cela modifier la nature même de l'extrait. Ainsi, par exemple l'extrait obtenu peut être lyophilisé par toutes méthodes classiques de lyophilisation. On obtient ainsi une poudre qui peut être utilisée directement ou bien mélanger dans un solvant approprié avant utilisation.

Préférentiellement, selon l'invention on utilise un extrait aqueux et encore plus préférentiellement un extrait réalisé avec un solvant composé d'eau et de propylène glycol vendu sous le nom d'Herbasol® par la société Cosmetochem's.

La famille des Rosacées comprend 27 genres parmi lesquels on peut citer à titre d'exemple les genres Agrimonia, Amygdalus, Armeniaca, Cerasus, Malus, Mespilus, Persica, Pirus, Prunus, Rosa, Rubus.

Ainsi, l'extrait de Rosacées de l'invention est un extrait préparé à partir de matériel issu d'au moins un végétal appartenant à un genre choisi parmi Agrimonia, Amygdalus, Armeniaca, Cerasus, Malus, Mespilus, Persica, Pirus, Prunus, Rosa; Rubus.

Préférentiellement selon l'invention le végétal appartient au genre Rosa.

Le genre Rosa comporte plus de 1000 espèces parmi lesquelles on peut citer *Rosa alba, Rosa alpina, Rosa canina, Rosa cinnamonea, Rosa gallica, Rosa repens, Rosa rubrifolia, Rosa rubiginosa, Rosa sempervirens, Rosa spinosissima, Rosa stylosa, Rosa tomentosa ou encore Rosa villosa.*

Ainsi, l'extrait de végétal du genre Rosa de l'invention est un extrait préparé à partir de matériel issu d'au moins un végétal appartenant à une espèce choisie parmi *Rosa alba, Rosa alpina, Rosa canina, Rosa cinnamonea, Rosa gallica, Rosa repens, Rosa rubrifolia, Rosa rubiginosa, Rosa sempervirens, Rosa spinosissima, Rosa stylosa, Rosa tomentosa, Rosa villosa.*

Préférentiellement selon l'invention le végétal appartient à l'espèce *Rosa gallica.*

On a vu préalablement dans le texte des exemples de désordres associés à un excès de synthèse et/ou de libération de bradykinine.

L'invention a en outre pour objet l'utilisation, à titre de principe actif dans une composition d'une quantité efficace d'au moins un extrait d'un végétal de la famille des Rosacées, l'extrait ou la composition étant destinés à traiter les désordres physiopathologiques associés à un excès de synthèse et/ou de libération de bradykinine.

Ainsi, selon un aspect particulier, l'invention à pour objet l'utilisation, à titre de principe actif dans une composition d'une quantité efficace d'au moins un extrait d'un végétal de la famille des Rosacées, l'extrait ou la composition étant destinés à traiter la vasodilatation et l'augmentation de la perméabilité vasculaire, l'hypotension, la douleur, la prolifération du tissu conjonctif, l'inflammation, la chute des cheveux, les diarrhées, les rhinites allergiques la contraction des muscles lisses des tractus digestif et respiratoire et de l'utérus.

L'invention a plus particulièrement pour objet l'utilisation à titre de principe actif dans une composition d'une quantité efficace d'au moins un extrait d'un végétal de la famille des Rosacées, l'extrait ou la composition étant destinés à traiter la couperose, la télangiectasie, un rhynophyma ou à empêcher la formation de chéloïdes.

Quelque soit la forme de la composition selon l'invention, la quantité d'extrait de Rosacées utilisée dans la composition est bien entendu fonction de l'effet recherché et peut donc varier dans une large mesure.

Pour donner un ordre de grandeur, dans la composition selon l'invention,l'extrait d'au moins un végétal de la famille des Rosacées est en une quantité représentant de 0,01% à 30% du poids total de la composition et préférentiellement en une quantité représentant de 0,1% à 20% du poids total de la composition.

La composition selon l'invention peut être une composition à usage cosmétique ou pharmaceutique.
Quelque soit la forme de la composition selon l'invention dans laquelle au moins un extrait de Rosacées est utilisé, celle-ci peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

Les compositions selon l'invention peuvent également consister en des préparations solides constituant des savons ou des pains de nettoyage.

Les compositions peuvent aussi être conditionnées sous forme de composition pour aérosol comprenant également un agent propulseur sous pression.

La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention la composition peut associer au moins un extrait de Rosacées à d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle;
- les extraits végétaux ou d'origine microbienne.

La présente invention a en outre pour objet un procédé de traitement cosmétique en vue de diminuer l'effet irritant d'une composition cosmétique, caractérisé par le fait que l'on utilise par application sur la peau, sur les cheveux, et/ou sur les muqueuses, une composition cosmétique comprenant au moins un extrait de Rosacées telle que décrite ci-dessus.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les exemples et compositions suivants illustrent l'invention sans la limiter aucunement. Dans les compositions les proportions indiquées sont des pourcentages en poids.

### Exemple 1 : Activité biologique d'un extrait de Rosa gallica :

L'extrait utilisé dans les essais présentés ici est un extrait de *Rosa gallica* vendu sous le nom d'Herbasol® par la société COSMETOCHEM's.

### 1) Mesure de l'affinité réceptorielle:

La mesure de l'affinité réceptorielle de l'extrait pour le récepteur B₂ de la bradykinine a été effectuée selon la méthode décrite par Jong, Y.J.L. et al, PNAS-USA 90, 1993, 10994.

Lors de chaque étude, la molécule de référence du récepteur étudié (NPC567) est testée à 8 concentrations (n=2) pour l'obtention d'une courbe standard permettant de valider l'expérimentation.

On obtient ainsi :
30 % de fixation pour l'extrait à 1 %
74 % de fixation pour l'extrait à 5 %
84 % de fixation pour l'extrait à 10 %

Les résultats de cette expérience mettent en évidence une affinité de l'extrait de *Rosa gallica* pour le récepteur à la bradykinine B₂ humain à des concentrations inférieures à 1 %.
La valeur de l'lC₅₀ est proche de 1%.

### 2) Mesure de l'activité antagoniste de la bradykinine :

Un test fonctionnel *ex vivo* réalisé sur les récepteurs à la bradykinine de type B₂ présents sur la veine jugulaire isolée de Lapin est réalisé pour mettre en évidence le caractère antagoniste de bradykinine, type B₂.
Les expériences *ex vivo* sont réalisées selon la méthode de Rhaleb, N.E. et al décrite dans Brit. J. Pharmacol., 99, 1990,445.

Après installation dans des cuves expérimentales, les tissus (muscles lisses) sont soumis à une tension initiale de 1 g. Une période d'équilibration d'au moins 60 minutes, au cours de laquelle la solution physiologique est plusieurs fois renouvelée et la tension initiale réajustée, est ensuite respectée avant l'addition de l'extrait.

Les expériences sont conduites en présence continue d'atropine (3.10⁻⁶ M), de pyrilamine (3.10⁻⁶ M) et d'indométhacine (10-⁶ M) pour s'affranchir des effets indirects de médiateurs mis en jeu lors de la stimulation d'autres types de récepteurs présents sur ce tissu.

Chaque préparation est initialement stimulée par un agoniste B2 à la concentration de 3.10⁻⁹ M qui induit des contractions. Il suffit ensuite de mettre dans le milieu un antagoniste spécifique B₂ pour voir disparaître ces contractions de manière dose-dépendante.

On obtient ainsi :
48 % de fixation pour l'extrait de l'exemple 1 à 0,5 %
79 % de fixation pour l'extrait de l'exemple 1 à 1 %
97 % de fixation pour l'extrait de l'exemple 1 à 5 %

Ces résultats montrent que l'extrait de Rosacées présente une activité antagoniste de bradykinine. Cette activité s'exprime préférentiellement, sur le récepteur B2.

### Exemple 2:

Exemples de formulations illustrant l'invention et particulièrement les compositions selon l'invention associant au moins un extrait de pétales de Rosacées et un produit à effet irritant. Ces compositions ont été obtenues par simple mélange des différents composants.

| Composition 1 : Lotion | |
|---|---|
| Herbasol® | 1,00 |
| Antioxydant | 0,05 |
| Isopropanol | 40,00 |
| Conservateur | 0,30 |
| Eau | qsp 100 % |
| | |

| Composition 2 : Gel pour le soin | |
|---|---|
| Herbasol® | 4,00 |
| Hydroxypropylcellulose^{*} | 1,00 |
| Antioxydant | 0,05 |
| Isopropanol | 40,00 |
| Conservateur | 0,30 |
| Eau | qsp 100 % |
| | |

| Composition 3 : Crème de soin (émulsion huile dans eau) | |
|---|---|
| Herbasol® | 5,00 |
| Stéarate de glycérol | 2,00 |
| Polysorbate 60^{**} | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |
| | |

| Composition 4 : Shampooing | |
|---|---|
| Herbasol® | 2,00 |
| Hydroxypropylcellulose^{*} | 1,00 |
| Parfum | 0,50 |
| Conservateur | 0,30 |
| Eau | qsp 100 % |
| | |

| Composition 5 : Crème de soin (émulsion huile/eau) | |
|---|---|
| Herbasol® | 4,00 |
| Stéarate de glycérol | 2,00 |
| Polysorbate 60^{**} | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |
| | |

| Composition 6 : Gel anti-douleur | |
|---|---|
| Herbasol® | 10,00 |
| Hydroxypropylcellulose^{*} | 1,00 |
| Antioxydant | 0,05 |
| Chlorhydrate de lidocaïne | 2,00 |
| Isopropanol | 40,00 |
| Conservateur | 0,30 |
| Eau | qsp 100 % |
| | |

| Composition 7 : Crème de soin de l'érythème solaire (émulsion huile-dans-eau) | |
|---|---|
| Herbasol® | 2,00 |
| Stéarate de glycérol | 2,00 |
| Polysorbate 60^{**} | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100 % |
| | |

| Composition 8 : Gel pour le traitement de l'acné | |
|---|---|
| Herbasol® | 8,00 |
| Acide tout trans rétinoïque | 0,05 |
| Hydroxypropylcellulose^{*} | 1,00 |
| Antioxydant | 0,05 |
| Isopropanol | 40,00 |
| Conservateur | 0,30 |
| Eau | qsp 100 % |
| | |

| Composition 9 : Lotion pour éliminer les cicatrices dues à l'acné | |
|---|---|
| Herbasol® | 8,00 |
| Acide glycolique | 50,00 |
| Hydroxypropylcellulose^{*} | 0,05 |
| NaOH | qsp pH = 2,8 |
| Ethanol | qsp 100 % |
| Conservateur | 0,30 |

| | |
|---|---|
| * : Klucel H® vendu par la société Hercules | |
| ** : Tween 60® vendu par la société lCl | |

## Revendications

1. Utilisation cosmétique, à titre de principe actif, dans une composition d'une quantité efficace d'au moins un extrait d'au moins un végétal de la famille des Rosacées en tant qu'antagoniste de bradykinine.

2. Utilisation d'une quantité efficace d'au moins un extrait d'au moins un végétal de la famille des Rosacées, pour la préparation d'une composition destinée à traiter les désordres associés à un excès de synthèse et/ou de libération bradykinine.

3. Utilisation d'une quantité efficace d'au moins un extrait d'un végétal de la famille des Rosacées, selon la revendication 2, la composition étant destinée à traiter la vasodilatation et l'augmentation de la perméabilité vasculaire, l'hypotension, la douleur, la prolifération du tissu conjonctif, l'inflammation, la chute des cheveux, les diarrhées, les rhinites allergiques la contraction des muscles lisses des tractus digestif et respiratoire et de l'utérus.

4. Utilisation d'une quantité efficace d'au moins un extrait d'un végétal de la famille des Rosacéesselon la revendication 2, la composition étant destinée à traiter la couperose, la télangiectasie, un rhynophyma ou à empêcher la formation de chéloïdes

5. Utilisation selon l'une quelconque des revendications 1 à 4, **caractérisée par le fait que** la composition est à usage cosmétique ou pharmaceutique.

6. Utilisation selon l'une quelconque des revendications 1 à 5, **caractérisé par le fait que** l'extrait d'au moins un végétal de la famille des Rosacées est en une quantité représentant de 0,01% à 30% du poids total de la composition et préférentiellement en une quantité représentant de 0,1% à 20% du poids total de la composition.

7. Utilisation selon l'une quelconque des revendications 1 à 6, **caractérisée par le fait que** l'extrait d'au moins un végétal de la famille des Rosacées est obtenu à partir de matériel végétal issu de plante entière ou de feuilles, de tiges, de fleurs, de pétales, de racines ou encore de cellules indifférenciées.

8. Utilisation selon la revendication 7, **caractérisée par le fait que** l'extrait d'au moins un végétal de la famille des Rosacées est obtenu à partir de pétales.

9. Utilisation selon l'une quelconque des revendications 1 à 8, **caractérisée par le fait que** l'extrait d'au moins un végétal de la famille des Rosacées est obtenu à partir de matériel végétal issu d'au moins un végétal de la famille des Rosacées cultivé *in vivo.*

10. Utilisation selon l'une quelconque des revendications 1 à 9, **caractérisée par le fait que** l'extrait est un extrait préparé à partir de matériel issu d'au moins un végétal appartenant à un genre choisi parmi Agrimonia, Amygdalus, Armeniaca, Cerasus, Malus, Mespilus, Persica, Pirus, Prunus, Rosa, Rubus.

11. Utilisation selon l'une quelconque des revendications 1 à 10, **caractérisée par le fait que** le végétal appartient au genre Rosa.

12. Utilisation selon l'une quelconque des revendications 1 à 11, **caractérisée par le fait que** l'extrait est un extrait préparé à partir de matériel issu d'au moins un végétal appartenant à une espèce choisie parmi *Rosa alba, Rosa alpina, Rosa canina, Rosa cinnamonea, Rosa gallica, Rosa repens, Rosa rubrifolia, Rosa rubiginosa, Rosa sempervirens, Rosa spinosissima, Rosa stylosa, Rosa tomentosa, Rosa villosa.*

13. Utilisation selon l'une quelconque des revendications 1 à 12, **caractérisée par le fait que** le végétal appartient à l'espèce *Rosa gallica.*

## Patentansprüche

1. Kosmetische Verwendung als Wirkstoff in einer Zusammensetzung einer wirksamen Menge mindestens eines Extraktes mindestens einer Pflanze aus der Familie der Rosaceae als Bradykinin-Antagonist.

2. Verwendung einer wirksamen Menge mindestens eines Extraktes mindestens einer Pflanze aus der Familie der Rosaceae zur Herstellung einer Zusammensetzung, die dazu vorgesehen ist, Störungen zu behandeln, die mit einer überschüssigen Synthese und/oder Freisetzung von Bradykinin verbunden sind.

3. Verwendung einer wirksamen Menge mindestens eines Extraktes einer Pflanze aus der Familie der Rosaceae nach Anspruch 2, wobei die Zusammensetzung dazu vorgesehen ist, Vasodilatation, die Erhöhung der vaskulären Permeabilität, niedrigen Blutdruck, Schmerz, Proliferation des Bindegewebes, Entzündungen, Haarausfall, Diarrhoe, allergische Rhinitis und die Kontraktion der glatten Muskulatur des Verdauungstrakts, des Atmungssystems und des Uterus zu behandeln.

4. Verwendung einer wirksamen Menge mindestens eines Extraktes einer Pflanze aus der Familie der Rosaceae nach Anspruch 2, wobei die Zusammensetzung dazu vorgesehen ist, Acne rosaceae, Teleangiektasie und Rhinophyma zu behandeln oder die Bildung von Keloiden zu verhindern.

5. Verwendung nach einem der Ansprüche 1 bis 4, **dadurch gekennzeichnet, dass** die Zusammensetzung zur kosmetischen oder pharmazeutischen Verwendung dienen soll.

6. Verwendung nach einem der Ansprüche 1 bis 5, **dadurch gekennzeichnet, dass** der Extrakt mindestens einer Pflanze aus der Familie der Rosaceae in einer Menge von 0,01 bis 30 Gew.-% des Gesamtgewichts der Zusammensetzung und vorzugsweise in einer Menge von 0, 1 bis 20 % des Gesamtgewichts der Zusammensetzung vorliegt.

7. Verwendung nach einem der Ansprüche 1 bis 6, **dadurch gekennzeichnet, dass** der Extrakt mindestens einer Pflanze aus der Familie der Rosaceae ausgehend von Pflanzenmaterial hergestellt wird, das von der ganzen Pflanzen oder den Blättern, Stängeln, Blüten, Blütenblättern oder Wurzeln oder aus undifferenzierten Zellen stammt.

8. Verwendung nach Anspruch 7, **dadurch gekennzeichnet, dass** der Extrakt mindestens einer Pflanze aus der Familie der Rosaceae ausgehend von Blütenblättern hergestellt wird.

9. Verwendung nach einem der Ansprüche 1 bis 8, **dadurch gekennzeichnet, dass** der Extrakt mindestens einer Pflanze aus der Familie der Rosaceae ausgehend von Pflanzenmaterial hergestellt wird, das von mindestens einer *in-vivo* kultivierten Pflanze aus der Familie Rosaceae stammt.

10. Verwendung nach einem der Ansprüche 1 bis 9, **dadurch gekennzeichnet, dass** der Extrakt ein Extrakt ist, der aus Pflanzenmaterial hergestellt wird, das von mindestens einer Pflanze aus einer Gattung stammt, die unter Agrimonia, Amygdalus, Armeniaca, Cerasus, Malus, Mespilus, Persica, Pyrus, Prunus, Rosa und Rubus ausgewählt ist.

11. Verwendung nach einem der Ansprüche 1 bis 10, **dadurch gekennzeichnet, dass** die Pflanze aus der Gattung Rosa stammt.

12. Verwendung nach einem der Ansprüche 1 bis 11, **dadurch gekennzeichnet, dass** der Extrakt ein Extrakt ist, der aus Pflanzenmaterial hergestellt wird, das von mindestens einer Pflanze einer Spezies stammt, die unter *Rosa alba, Rosa alpina, Rosa canina, Rosa cinnamonea, Rosa gallica, Rosa repens, Rosa rubrifolia, Rosa rubiginosa, Rosa sempervirens, Rosa spinosissima, Rosa stylosa, Rosa tomentosa* und *Rosa villosa* ausgewählt ist.

13. Verwendung nach einem der Ansprüche 1 bis 12, **dadurch gekennzeichnet, dass** die Pflanze aus der Spezies *Rosa gallica* stammt.

## Claims

1. Cosmetic use, as active ingredient, in a composition, of an effective quantity of at least one extract of at least one plant of the Rosaceae family as bradykinin antagonist.

2. Use of an effective quantity of at least one extract of at least one plant of the Rosaceae family, for the preparation of a composition intended for treating disorders associated with an excessive synthesis and/or release of bradykinin.

3. Use of an effective quantity of at least one extract of a plant of the Rosaceae family, according to Claim 2, the composition being intended for treating vasodilation and increase in vascular permeability, hypotension, pain, proliferation of the connective tissue, inflammation, hair loss, diarrhoeas, allergic rhinitis, the contraction of the smooth muscles of the digestive and respiratory tracts and of the uterus.

4. Use of an effective quantity of at least one extract of a plant of the Rosaceae family according to Claim 2, the composition being intended for treating acne rosacea, telangiectasia, a rhynophyma or for preventing the formation of keloids.

5. Use according to any one of Claims 1 to 4, **characterized in that** the composition is for cosmetic or pharmaceutical use.

6. Use according to any one of Claims 1 to 5, **characterized in that** the extract of at least one plant of the Rosaceae family is in a quantity representing from 0.01% to 30% of the total weight of the composition and preferably in a quantity representing from 0.1% to 20% of the total weight of the composition.

7. Use according to any one of Claims 1 to 6, **characterized in that** the extract of at least one plant of the Rosaceae family is obtained from plant material derived from a whole plant or from leaves, stems, flowers, petals, roots or alternatively from undifferentiated cells.

8. Use according to Claim 7, **characterized in that** the extract of at least one plant of the Rosaceae family is obtained from petals.

9. Use according to any one of Claims 1 to 8, **characterized in that** the extract of at least one plant of the Rosaceae family is obtained from plant material derived from at least one plant of the Rosaceae family cultured *in vivo*.

10. Use according to any one of Claims 1 to 9, **characterized in that** the extract is an extract prepared from material derived from at least one plant belonging to a genus chosen from Agrimonia, Amygdalus, Armeniaca, Cerasus, Malus, Mespilus, Persica, Pirus, Prunus, Rosa, Rubus.

11. Use according to any one of Claims 1 to 10, **characterized in that** the plant belongs to the Rosa genus.

12. Use according to any one of Claims 1 to 11, **characterized in that** the extract is an extract prepared from material derived from at least one plant belonging to a species chosen from *Rosa alba, Rosa alpina, Rosa canina, Rosa cinnamonea, Rosa gallica, Rosa repens, Rosa rubrifolia, Rosa rubiginosa, Rosa sempervirens, Rosa spinosissima, Rosa stylosa, Rosa tomentosa, Rosa villosa.*

13. Use according to any one of Claims 1 to 12, **characterized in that** the plant belongs to the species *Rosa gallica.*
